(19) Europäisches Patentamt
European Patent Office
Office européen des brevets

(11) Veröffentlichungsnummer : **0 394 840 B1**

(12) **EUROPÄISCHE PATENTSCHRIFT**

(45) Veröffentlichungstag der Patentschrift :
**03.11.93 Patentblatt 93/44**

(51) Int. Cl.$^5$ : **C07C 409/04,** C07C 409/06, C07C 409/08, C07C 407/00

(21) Anmeldenummer : **90107433.6**

(22) Anmeldetag : **19.04.90**

(54) **4- Hydroperoxi-2-alkensäuren und deren Ester.**

(30) Priorität : **22.04.89 DE 3913330**

(43) Veröffentlichungstag der Anmeldung :
**31.10.90 Patentblatt 90/44**

(45) Bekanntmachung des Hinweises auf die Patenterteilung :
**03.11.93 Patentblatt 93/44**

(84) Benannte Vertragsstaaten :
**AT BE DE FR GB IT NL**

(56) Entgegenhaltungen :
**FR-A- 2 079 157**
**SYNTHESIS, Band 12, 1986, Seiten 1050-1052; W. ADAM et al.: "Regioselective Synthesis of 2-Hydroperoxy-2-methylene-butanoic Acid Derivatives via Photooxygenation of Tiglic Acid Derivatives"**

(73) Patentinhaber : **BASF Aktiengesellschaft**
**Carl-Bosch-Strasse 38**
**D-67063 Ludwigshafen (DE)**

(72) Erfinder : **Rieber, Norbert, Dr.**
**Liebfrauenstr. 1c**
**D - 6800 Mannheim (DE)**

EP 0 394 840 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 4-Hydroperoxi-2-alkensäuren und deren Ester sowie ein Verfahren zu deren Herstellung.

Der vorliegenden Erfindung lag die Aufgabe zugrunde, neue Zwischenprodukte zu finden, die die Herstellung von 3,4-Epoxialkansäuren und/oder 4-Hydroxi-2-alkensäuren ermöglichen.

Demgemäß wurden die neuen 4-Hydroperoxi-2-alkensäuren und deren Ester der allgemeinen Formel I

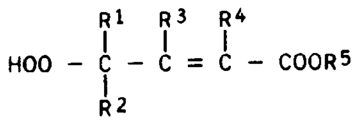

(I),

in der

$R^1$, $R^2$, $R^3$, $R^4$, $R^5$ Wasserstoff oder einen acyclischen, cyclischen oder unverzweigten Alkyl- oder Aralkylrest mit 1 bis 30 Kohlenstoffatomen bedeuten,

gefunden sowie ein Verfahren zu deren Herstellung.

Die Substituenten $R^1$, $R^2$, $R^3$, $R^4$ und $R^5$ der 4-Hydroperoxi-2-alkensäuren und deren Ester haben unabhängig voneinander folgende Bedeutungen:

- Wasserstoff oder einen acyclischen, cyclischen, verzweigten oder unverzweigten Alkyl- oder Aralkylrest mit 1 bis 30 Kohlenstoffatomen, im speziellen
- Wasserstoff,
- unverzweigtes oder verzweigtes $C_1$-$C_{18}$-Alkyl, bevorzugt $C_1$-$C_8$-Alkyl, besonders bevorzugt $C_1$-$C_8$-Alkyl wie Methyl, Ethyl, n-Propyl, iso-Propyl, n-Butyl, iso-Butyl, sec.-Butyl und tert.-Butyl,
- $C_3$-$C_{12}$-Cycloalkyl, bevorzugt $C_3$-$C_8$-Cycloalkyl wie Cyclopropyl, Cyclobutyl, Cyclopentyl, Cyclohexyl, Cycloheptyl und Cyclooctyl, besonders bevorzugt Cyclopentyl und Cyclohexyl,
- $C_4$-$C_{18}$-Cycloalkyl-alkyl, bevorzugt $C_4$-$C_{12}$-Cycloalkyl-alkyl, besonders bevorzugt $C_5$-$C_{10}$-Cycloalkyl-alkyl wie Cyclohexyl-methyl, 1-Cyclohexyl-ethyl und 2-Cyclohexyl-ethyl,
- $C_4$-$C_{18}$-Alkylcycloalkyl, bevorzugt $C_4$-$C_{12}$-Alkylcycloalkyl, besonders bevorzugt $C_5$-$C_{10}$-Alkylcycloalkyl wie 2-Methylcyclopentyl, 3-Methylcyclopentyl, 2-Methylcyclohexyl, 3-Methylcyclohexyl, 4-Methylcyclohexyl und 2,4-Dimethylcyclohexyl,
- $C_5$-$C_{18}$-Alkylcycloalkyl-alkyl, bevorzugt $C_5$-$C_{12}$-Alkylcycloalkyl-alkyl, besonders bevorzugt $C_6$-$C_{10}$-Alkylcycloalkyl-alkyl wie 2-Methylcyclopentyl-methyl und 2-Methylcyclohexyl-methyl,
- $C_7$-$C_{18}$-Aralkyl, bevorzugt $C_7$-$C_{14}$-Aralkyl, besonders bevorzugt $C_7$-$C_{10}$-Aralkyl wie Benzyl, 1-Phenethyl und 2-Phenethyl.

Der Substituent $R^1$ bedeutet vorzugsweise Wasserstoff oder $C_1$-$C_4$-Alkyl, $R^2$ vorzugsweise Wasserstoff oder Methyl, $R^3$ vorzugsweise Wasserstoff oder Methyl, $R^4$ vorzugsweise Wasserstoff oder Methyl und $R^5$ vorzugsweise Wasserstoff oder $C_1$-$C_{12}$-Alkyl, besonders bevorzugt $C_1$-$C_8$-Alkyl wie Methyl, Ethyl, n-Propyl, iso-Propyl, n-Butyl, iso-Butyl, sec.-Butyl, tert.-Butyl, Pentyl, Hexyl, Heptyl oder Octyl.

Bevorzugte 4-Hydroperoxi-2-alkensäuren oder 4-Hydroperoxi-2-alkensäureester I sind:

Vinylessigsäure
Vinylessigsäure-isobutylester
2-Methyl-3-butensäure
2-Methyl-3-butensäure-ethylester
3-Pentensäure
3-Pentensäure-methylester
3-Pentensäure-isopropylester
3-Pentensäure-n-hexylester
3-Pentensäure-sek.-butylester

Besonders bevorzugt wird 3-Pentensäure-methylester als Verbindungen I.

Die 4-Hydroperoxi-2-alkensäuren I sind nach folgenden Methoden erhältlich:

Die Umsetzung erfolgt durch Behandlung von 3-Alkensäuren oder deren Ester der allgemeinen Formel II mit Sauerstoff enthaltenden Gasen bei 20 bis 150°C und 0,01 bis 50 bar nach folgender Reaktionsgleichung:

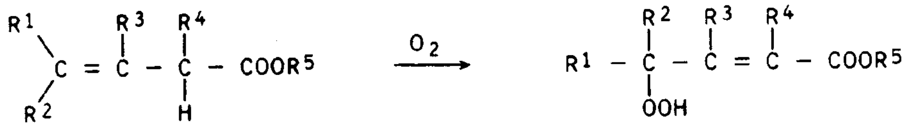

(II) (I)

Statt der zu erwartenden 2-Hydroperoxi-3-alkensäuren und deren Ester der allgemeinen Formel III

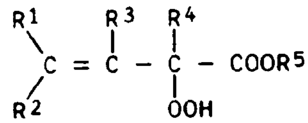

(III)

entstehen bei der Oxidation unter Doppelbindungsverschiebung die 4-Hydroperoxi-2-alkensäuren und deren Ester I.

Die Reaktion kann diskontinuierlich oder kontinuierlich z.B. in Blasensäulen oder Rührkesseln unter Durchleiten von Sauerstoff enthaltenden Gasen durch Fritten oder Einleitungsrohre bei 20 bis 150°C und 0,01 bis 50 bar, vorzugsweise bei 40 bis 120°C und 0,1 bis 5 bar, besonders bevorzugt bei 50 bis 100°C und Atmosphärendruck durchgeführt werden.

Die Reaktion läßt sich vorzugsweise unter Zusatz von 0,001 bis 1,5 Gew.% eines Radikalbildners, wie Azo-bis-isobutyronitril oder Dibenzoylperoxid und/oder 0,001 bis 50 Gew.%, basischer Verbindungen, wie Alkali- und Erdalkalihydrogencarbonaten, z.B. $NaHCO_3$, $KHCO_3$ und $CaHCO_3$, Alkali- und Erdalkalicarbonaten, wie $Na_2CO_3$, $K_2CO_3$ und $CaCO_3$, durchführen.

Als Sauerstoff enthaltende Gase eignen sich vorzugsweise Luft, Sauerstoff-Stickstoff-Gemische oder reiner Sauerstoff.

Die 3-Alkensäuren und deren Ester II sind allgemein bekannt oder können nach DE-A-2 630 086, Organic Syntheses, Coll. Vol. III, Seite 851 (1955) oder Tetrahedron Lett. 23, 3901 (1982) hergestellt werden.

Die neuen 4-Hydroperoxi-2-alkensäuren und deren Ester I eignen sich als Zwischenprodukte zur Herstellung von 4-Ketocarbonsäuren und deren Ester insbesondere Methylester (über die Zwischenstufen 3,4-Epoxivaleriansäuremethylester und 4-Hydroxi-2-pentensäuremethylester), die als Weichmacher für Kunststoffe oder bei der Herstellung von pharmazeutischen Wirkstoffen Verwendung finden.

Beispiele

Beispiel 1

Herstellung der 4-Hydroperoxi-2-pentensäureethylester

180 g 3-Pentensäureethylester werden unter Zusatz von 0,5 g Porofor N® in einer Blasensäule 7 Stunden mit 2 l $O_2$/h bei 75 bis 85°C oxidiert. Nach dem Abkühlen auf R.T. wird 2 x mit je 50 ml 15 %iger wäßriger Natriumcarbonat-Lösung extrahiert, die organische Phase mit Natrium-sulfat getrocknet und i. Vak. bei 40°C/20 mbar eingeengt. Nach Strippung mit Stickstoff bei 20°C/1 mbar erhält man 13 g (79 %) 4-Hydroperoxi-2-pentencarbonsäureethylester als Öl ($n_D^{20}$ = 1,4581, 79%ig nach Peroxid-Titration).

Entsprechend Beispiel 1 wurden die in der folgenden Tabelle 1 zusammengefaßten Hydroperoxide hergestellt:

| Beispiel Nr. | $R^1$ | $R^2$ | $R^3$ | $R^4$ | $R^5$ | Hydroperoxid-Konzentation [%] | $n_D^{20}$ |
|---|---|---|---|---|---|---|---|
| 2 | H | H | H | H | $(CH_3)_2CH-CH_2-$ | 68 | 1,4595 |
| 3 | H | H | H | $CH_3$ | $C_2H_5$ | 75 | 1,4667 |
| 4 | $CH_3$ | H | H | H | $CH_3-$ | 77 | 1,4625 |
| 5 | $CH_3$ | H | H | H | $(CH_3)_2CH-$ | 74 | 1,4520 |
| 6 | $CH_3$ | H | H | H | $CH_3-(CH_2)_5-$ | 66 | 1,4548 |
| 7 | $CH_3$ | H | H | H | $C_2H_5CH(CH_3)-$ | 71 | 1,4530 |

## Patentansprüche

1. 4-Hydroperoxi-2-alkensäuren und deren Ester der allgemeinen Formel I

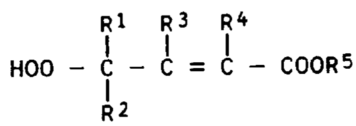

(I),

in der

$R^1$, $R^2$, $R^3$, $R^4$, $R^5$ Wasserstoff oder einen acyclischen, cyclischen oder unverzweigten Alkyl- oder Aralkylrest mit 1 bis 30 Kohlenstoffatomen bedeuten.

2. 4-Hydroperoxi-2-alkensäuren und deren Ester I nach Anspruch 1, in der

$R^1$, $R^2$, $R^3$, $R^4$, $R^5$ Wasserstoff, $C_1$-$C_{20}$-Alkyl, $C_3$-$C_{12}$-Cycloalkyl, $C_4$-$C_{20}$-Cycloalkyl-alkyl, $C_4$-$C_{20}$-Alkylcycloalkyl, $C_5$-$C_{30}$-Alkylcycloalkyl-alkyl oder $C_7$-$C_{20}$-Aralkyl bedeuten.

3. Verfahren zur Herstellung von 4-Hydroperoxi-2-alkensäuren und deren Ester I nach Anspruch 1, dadurch gekennzeichnet, daß man 3-Alkensäuren oder deren Ester der allgemeinen Formel II

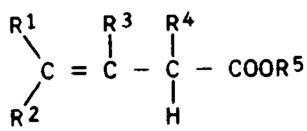

(II),

in der $R^1$ bis $R^5$ die für die Verbindungen I genannten Bedeutungen haben, mit Sauerstoff enthaltenden Gasen bei 20 bis 150°C und 0,01 bis 50 bar umsetzt.

## Claims

1. A 4-hydroperoxy-2-alkenoic acid or its ester of the formula I

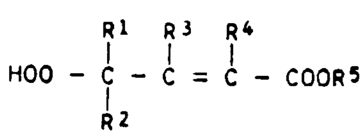

(I),

where

$R^1$, $R^2$, $R^3$, $R^4$ and $R^5$ are each hydrogen or acyclic, cyclic or unbranched alkyl or aralkyl of 1-30 carbons.

**2.** A 4-hydroperoxy-2-alkenoic acid or its ester I as claimed in claim 1, where $R^1$, $R^2$, $R^3$, $R^4$ and $R^5$ are each hydrogen, $C_1$-$C_{20}$-alkyl, $C_3$-$C_{12}$-cycloalkyl, $C_4$-$C_{20}$-cycloalkylalkyl, $C_4$-$C_{20}$-alkylcycloalkyl, $C_5$-$C_{30}$-alkylcycloalkylalkyl or $C_7$-$C_{20}$-aralkyl.

**3.** A process for preparing 4-hydroperoxy-2-alkenoic acids and their esters I as claimed in claim 1, which comprises reacting 3-alkenoic acids or their esters of the formula II

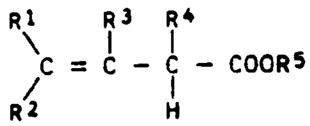 (II),

where $R^1$ to $R^5$ have the meanings specified for compounds I, with oxygen containing gases at 20-150°C under 0.01-50 bar.

**Revendications**

**1.** Acides 4-hydroperoxy-2-alcénoïques et leurs esters de formule générale I

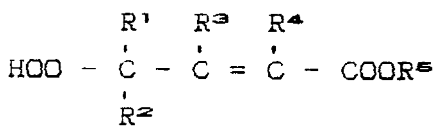 (I)

dans laquelle

| | |
|---|---|
| $R^1$, $R^2$, $R^3$, $R^4$, $R^5$ | représentent chacun un atome d'hydrogène ou un reste alkyle ou aralkyle acyclique, cyclique ou non ramifié à 1-30 atomes de carbone. |

**2.** Acides 4-hydroperoxy-2-alcénoïques et leurs esters I selon la revendication 1, dans lesquels

| | |
|---|---|
| $R^1$, $R^2$, $R^3$, $R^4$, $R^5$ | représentent chacun un atome d'hydrogène ou un reste alkyle en $C_1$-$C_{20}$, cycloalkyle en $C_3$-$C_{12}$, cycloalkyl-alkyle en $C_4$-$C_{20}$, alkylcycloalkyle en $C_4$-$C_{20}$, alkylcycloalkyl-alkyle en $C_5$-$C_{30}$, ou aralkyle en $C_7$-$C_{20}$. |

**3.** Procédé de préparation d'acides 4-hydroperoxy-2-alcénoiques et de leurs esters I selon la revendication 1, caractérisé en ce qu'on fait réagir des acides 3-alcénoïques ou leurs esters de formule générale II

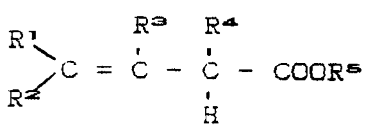 (II)

dans laquelle $R^1$ à $R^5$ ont les significations indiquées à propos des composés I, avec des gaz contenant de l'oxygène, à une température de 20 à 150°C et sous une pression de 0,01 à 50 bar.